# EUROPEAN PATENT APPLICATION

(11) **EP 1 679 066 A1**
(43) Date of publication of application: **12.07.2006**
(21) Application number: 04793297.5
(22) Date of filing: 26.10.2004
(51) Int. Cl.: A61K 9/50, A61K 47/38, A61K 47/32, A61K 47/34, A61K 47/36

(54) **DRUG-CONTAINING COATED MICROPARTICLE FOR ORALLY DISINTEGRATING TABLET**

(30) Priority: 27.10.2003 US 515070 P
(71) Applicant: Astellas Pharma Inc., Chuo-ku, Tokyo (JP)
(72) Inventor: KURIMOTO, Ippei, c/o Astellas Pharma Inc., Chuo-ku, Tokyo 103-8411 (JP); KASASHIMA, Yuki, c/o Astellas Pharma Inc., Chuo-ku, Tokyo 103-8411 (JP); KAWAI, Hitoshi, c/o Astellas Pharma Inc., Chuo-ku, Tokyo 103- 8411 (JP); TAKAISHI, Yuuki, c/o Astellas Pharma Inc., Chuo-ku, Tokyo 103-8411 (JP); KATSUMA, Masataka, c/o Astellas Pharma Inc., Chuo-ku, Tokyo 103-8411 (JP); OHI, Hiroshi, c/o Astellas Pharma Inc., Chuo-ku, Tokyo 103-8411 (JP); YOSHIDA, Takayuki, c/o Astellas Pharma Inc., Chuo-ku, Tokyo 103-8411 (JP); TASAKI, Hiroaki,c/o Astellas Pharma Inc., Chuo-ku, Tokyo 103-8411 (JP)
(74) Representative: HOFFMANN EITLE
(86) International application number: PCT/JP2004/016196
(87) International publication number: WO 2005/039542

(57) **Abstract**

The present invention makes it possible to provide drug-containing coated microparticles for quick-disintegrating oral tablets wherein microparticles containing a drug with an unpleasant taste are coated with a film composed of (1) a pH-independent water-insoluble polymer accounting for 60% or more but less than 80% of the film and (2) a pH-independent water-soluble substance accounting for more than 20% to 40% or less of the film, these microparticles being **characterized in that** the rate of dissolution of the drug from the drug-containing microparticles is 0% to 10% in one minute and 80% to 100% in 30 minutes, and the average particle diameter is 350 µm or less, in order to realize sufficient control of oral drug dissolution and fast gastrointestinal drug dissolution.

## Description

### Technical Field

The present invention relates to drug-containing coated microparticles for quick-disintegrating oral tablets wherein microparticles containing an unpleasant-tasting drug are coated with a film composed of
(1) a pH-independent water-insoluble polymer accounting for 60% or more but less than 80% of the film and
(2) a pH-independent water-soluble substance accounting for more than 20% to 40% or less of the film,
these microparticles being characterized in that the rate of dissolution of the drug from the drug-containing microparticles is 0% to 10% in one minute and 80% to 100% in 30 minutes, and the average particle diameter is 350 µm or less.

### Prior Art

Quick-disintegrating oral tablets can be easily taken by patients who have difficulty swallowing and they can be easily taken without water. This dosage form has recently received attention because of this convenience. Nevertheless, when a drug with an unpleasant taste, particularly a very bitter taste, is made into quick-disintegrating oral tablets, product value is greatly compromised because of this unpleasant taste. That is, the concentration with which the bitter taste is noticed, that is, the threshold value, is low with drugs that have a strong bitter taste; therefore, a bitter taste is noticed, even if only a very small amount of the drug remains in the oral cavity. Consequently, when a drug with a strong bitter taste is used for quick-disintegrating oral.tablets, the time for which the bitter taste is noticed appears to be longer than with drugs that do not have a strong bitter taste, and this affects drug compliance.

There are many drugs with an unpleasant taste, particularly a strong bitter taste. When these drugs are being considered for pharmaceutical preparations, a method appropriate for degree of bitter taste of the drug is generally selected. Specifically, there are methods whereby a flavoring, sweetener, or other agent is added, film coating methods whereby a polymer base is used, and other methods. Methods whereby a flavoring or sweetener is added are convenient methods because the flavoring or sweetener can be simply mixed with an unpleasant-tasting drug during preparation. However, these methods alone often do not sufficiently mask the bitter taste of the drug and they can only be used for a very limited number of drugs with a weak unpleasant taste.

Assuming that the film coating will be used for quick-disintegrating oral tablets, the film coating method that uses a polymer base is specifically a method whereby microparticles containing a drug are prepared and these microparticles are film coated, but this method is selected for the purpose of controlling drug dissolution to a short time only. The microparticles that are used are 350 µm or smaller, which is a much different size than the 500 µm that is used in ordinary preparations. Dissolution of the drug is affected by the particle surface area, and the like; therefore, it is difficult to apply coating technology for ordinary preparations to quick-disintegrating oral tablets without further modification.

Moreover, quick-disintegrating oral tablets disintegrate in the oral cavity instantly and it is therefore necessary to sufficiently control drug dissolution in the early phase after administration; that is, it is necessary to control drug dissolution in the early phase after administration more precisely than with ordinary preparations.

Quick-disintegrating oral tablets are often positioned as a dosage form that improves the compliance that is obtained with ordinary tablets, which were marketed before quick-disintegrating oral tablets, and of course controlling the instantly unpleasant taste, as well as guaranteeing bioavailability and guaranteeing biological equivalency of a quick-disintegrating oral tablets, are very important in terms of certifying the quality of the tablets. In terms of guaranteeing bioavailability, and the like, it is also necessary at the same time to guarantee quick dissolution of the drug once the preparation has moved from the oral cavity to the gastrointestinal tract.

Although they are not a technology for quick-disintegrating oral tablets, film coating methods that use a polymer base appropriate for granules, and the like have been known for years. For instance, JP (Kokai) S57-58631 discloses technology whereby reduction of the bitter taste of a drug and dissolution control are both accomplished by combining a water-insoluble polymer and an acid-soluble polymer or an enteric polymer. However, judging from the results in Table 1 and Figures 1 and 2 of the Specification, there is a phenomenon whereby dissolution fluctuates considerably with pH; therefore, there is a need for further improvement taking into consideration fluctuations in stomach pH of the patient taking the medication.

Moreover, JP (Kokai) H3-130214 (corresponding EP040925) discloses a fast-release pharmaceutical preparation wherein an unpleasant-tasting drug and a substance that swells when exposed to water are contained in at least the core, and the unpleasant taste of the drug is masked by coating this core with a coating film layer containing at least ethyl cellulose and a water-soluble substance. This technology intentionally accelerates the last half of drug dissolution using the substance contained in the core that swells when exposed to water. However, this technology presumes that water will also be taken, and judging from the time for which the unpleasant taste is masked in each of the working examples, the bitter taste of the drug is not controlled to the extent required with a quick-disintegrating oral tablets.

International Publication Pamphlet WO 98/30209 reports on the invention of a pharmaceutical dosage form that dissolves quickly and whose taste is masked wherein core particles with an average particle diameter of 80 to 400 µm are coated by a polymer film. This technology is a pharmaceutical dosage form consisting of core particles containing a drug, microcrystalline cellulose, and a low-substitution hydroxypropyl cellulose as the substance that swells; an inner coating layer of a water-soluble polymer intended to shield from the outer coating layer, and an outer coating layer containing an acid-soluble polymer that is insoluble in saliva. However, because an acid-soluble polymer is used for the outer coating layer, this is not a technology that can respond to pH fluctuations in the stomach, and the like of the patient. There is a need for stable drug dissolution that is unaffected by fluctuations in stomach pH of the patient.

Consequently, it is difficult to apply these technologies to quick-disintegrating oral tablets.

On the other hand, International Publication Pamphlet WO 02/96392 relates to reduction of the bitter taste of a very water-soluble drug and discloses an invention relating to drug microparticles that are film coated with a combination of a water-insoluble polymer and a water-soluble polymer. Ethyl cellulose, hydroxypropylmethyl cellulose phthalate, hydroxypropylmethyl cellulose succinate, and the like are used as the water-insoluble polymer of this invention. In light of the fact that pH inside the mouth is approximately neutral, the selection of an enteric base is undesirable because it allows the bitter taste to escape. An enteric base is often selected in this invention, but there is a concern that the bitter taste will not be sufficiently controlled.

Thus far there are no coated microparticles appropriate for controlling the bitter taste of various drugs with which both sufficient control of dissolution inside the mouth and fast dissolution after movement to the stomach are accomplished with an average particle diameter appropriate for quick-disintegrating oral tablets of 350 µm or smaller, using a combination of a water-insoluble polymer and a water-soluble substance unaffected by pH.

Consequently, the object of the present invention is to provide drug-containing coated microparticles for quick-disintegrating oral tablets with which the instantly unpleasant taste of a variety of unpleasant tasting drugs is sufficiently controlled and the drug quickly dissolves after movement to the digestive tract.

### Disclosure of the Invention

The inventors and others conducted intense studies of microparticles with which an unpleasant taste is reduced by a combination of a water-insoluble polymer and a water-soluble substance unaffected by pH in order to inhibit an instantly unpleasant taste and to obtain fast drug dissolution after movement to the stomach. Moreover, when the inventors and others simultaneously studied the criteria for inhibition of an unpleasant taste and fast drug dissolution after movement to the stomach, they discovered that it is necessary to control dissolution to 10% or less one minute after starting the dissolution test, or when the drug is in the oral cavity, and then maintain dissolution of 80% or higher 30 minutes after starting the dissolution test, which is once the drug has moved to the stomach. Furthermore, the inventors successfully completed the present invention upon discovering that the above-mentioned criteria are satisfied and the object of the present invention is accomplished with a variety of drugs when a specific ratio of water-insoluble polymer and water-soluble substance unaffected by pH is selected.

That is, the present invention relates to
1. drug-containing coated microparticles for quick-disintegrating oral tablets wherein microparticles containing a drug with an unpleasant taste are coated with a film composed of
   (1) a pH-independent water-insoluble polymer accounting for 60% or more but less than 80% of the film and
   (2) a pH-independent water-soluble substance accounting for more than 20% to 40% or less of the film,
   these microparticles being characterized in that the rate of dissolution of the drug from the drug-containing microparticles is 0% to 10% in one minute and 80% to 100% in 30 minutes, and the average particle diameter is 350 µm or less;
2. the drug-containing coated microparticles for quick-disintegrating oral tablets according to above-mentioned 1, characterized in that the pH-independent water-insoluble polymer is one or more selected from the group consisting of ethyl cellulose, aminoalkyl methacrylate copolymer RL, aminoalkyl methacrylate copolymer RS, and ethyl acrylate-methyl methacrylate copolymer;
3. the drug-containing coated microparticles for quick-disintegrating oral tablets according to above-mentioned 1, characterized in that the pH-independent water-soluble substance is one or more selected from the group consisting of hydroxypropylmethyl cellulose, hydroxypropyl cellulose, hydroxyethyl cellulose, polyvinyl pyrrolidone, polyethylene glycol, polyethylene oxide, lactose, sucrose, mannitol, and maltose; and
4. the quick-disintegrating oral tablets containing the drug-containing coated microparticles for quick-disintegrating oral tablets according to above-mentioned 1.

The "unpleasant taste" of the present invention means a taste that generates an unpleasant feeling when the tablets are taken, and specifically indicates a bitter, tart, acrid, astringent, or other taste.

The "water-insoluble polymer" used in the present invention is a pharmaceutically acceptable water-insoluble polymer with which the objects of the present invention can be accomplished using the structure of the present invention, and water-insoluble polymers that will not dissolve at any pH are cited. Specific examples are ethyl cellulose and other cellulose polymers, and aminoalkyl methacrylate copolymer RL (for instance, brand name; Eudragit RL, Rohm GmbH) aminoalkyl methacrylate copolymer RS (for instance, brand name; Eudragit RS, Rohm GmbH), ethyl acrylate-methyl methacrylate copolymer (for instance, brand name; Eudragit NE30D, Rohm GmbH), and other pH-independent acrylic polymers. Ethyl cellulose is preferred. Moreover, one or a combination of two or more water-insoluble polymers can be used.

The "pH-independent" means that there are no large fluctuations in solubility; regardless of pH.

The "water-soluble substance" used in the present invention is a pharmaceutically acceptable water-soluble substance with which the objects of the present invention can be accomplished using the structure of the present invention. Specific examples of this water-soluble substance are hyroxypropyl methyl cellulose, hydroxypropyl cellulose, hydroxyethyl cellulose, and other cellulose polymers; lactose, sucrose, mannitol, and other saccharides; and polyvinyl pyrrolidone, polyethylene glycol, polyethylene oxide, and other water-soluble polymers. Hydroxpropyl methyl cellulose and hydroxypropyl cellulose are preferred.

Moreover, one or a combination of two or more water-soluble substances can also be used.

The appropriate ratio for realizing the objects of the present invention is selected as the composition ratio of the water-insoluble polymer and water-soluble substance. Specifically, the ratio of water-insoluble polymer in the coating base that is used is 60 wt% or higher but less than 80 wt%. A ratio of 65 wt% or higher but less than 80 wt% is preferred, one of 70 wt% or higher but less than 80% is particularly preferred, and a ratio of 70 wt% to 75 wt% is ideal.

If the ratio of water-insoluble polymer is lower than 60 wt%, there is a concern that it will not be possible to sufficiently control drug dissolution in the oral cavity; therefore, a large amount of coating will be needed, which is not appropriate for actual production. On the other hand, if the amount of coating is 80 wt% or higher, there is the risk that fast gastrointestinal drug dissolution cannot be realized.

There are no restrictions to the amount of coating of film consisting of water-insoluble polymer and water-soluble substance and the amount of coating of film that is appropriate for realizing the object of the present invention is selected as this amount. Nevertheless, production time increases with an increase in the amount of coating; therefore, it is preferred that a small amount of coating for controlling an unpleasant taste. For instance, the amount of coating is 0.5 to 90 wt% for microparticles containing a drug whose unpleasant taste should be reduced. The amount of coating is preferably 1 to 80 wt%, particularly 1 to 50 wt%, ideally 1 to 25 wt%.

If the amount of coating is less than 0.5 wt%, it will not be possible to sufficiently control drug dissolution in the oral cavity.

The "control drug dissolution in the oral cavity" in the present invention means that the drug dissolution rate one minute after starting the dissolution test is 10% or less when a phosphate buffer with a pH of 6.8 is used (Japanese Pharmacopeia Disintegration Testing Methods Fluid No. 2; same hereafter). It is possible to sufficiently control the unpleasant taste of a drug by controlling drug dissolution to this dissolution rate or less.

The "fast dissolution after movement to the stomach" means that drug dissolution 30 minutes after starting the dissolution test is 80% or more when a hydrochloride buffer with a pH of 1.2 (Japanese Pharmacopeia Disintegration Testing Method, Fluid No. 1; same hereafter) or a phosphate buffer with a pH of 6.8 (Japanese Pharmacopeia Disintegration Testing Method, Fluid No. 2) is used. Selection of the test fluid involves, for instance, confirming whether or not solubility of the drug to be used is high near acidity or high near neutrality and selecting as the test fluid the solution with a pH at which drug solubility is low. This dissolution rate is the criterion that indicates that bioavailability is not diminished when a conventional preparation is administered and is the value established by the inventors.

Top spraying, side spraying, bottom spraying, and a variety of other types of film coating methods are selected in order to realize the drug-containing coated microparticles of the present invention, and of these, the side spraying-type film coating method is preferred. A dense film coating layer can be made with this method; as a result, the amount of film coating or the film coating layer can be thinner.

There are no special restrictions to the drug used in the present invention as long as it is an unpleasant tasting drug that is used as a medically active ingredient.

The present invention is appropriate for a variety of drugs. When attempting to reduce the unpleasant taste of the drug, the present invention is particularly effective for drugs with which it is difficult to simultaneously accomplish the opposing objectives of sufficient control of drug dissolution in the oral cavity and guaranteeing fast drug dissolution in gastrointestinal tract since an unpleasant-tasting drug is quickly dissolved in either the mouth or the gastrointestinal tract.

Examples of this drug are antidepressants, hypnotics and sedatives, sleep aids, anti-anxiety agents, anticonvulsants, anti-migraine drugs, antipyretic analgesic antiinflammatory drugs, anti-Parkinson's agents, psychoneurological agents, drugs used to treat dementia, other CNS drugs, musculoskeletal relaxants, autonomic nerve agents, antispasmodics, cardiotonics, agents for arrhythmia, diuretics, agents for hypertension, vasoconstrictors, vasodilators, drugs for the circulatory system, agents for hyperlipidemia, other drugs for the circulatory system, antitussive expectorants, bronchodilators, other drugs used to treat allergies, antidiarrheal agents, agents for controlling intestinal function, agents for peptic ulcers, digestives, antacids, other drugs for treating the gastrointestinal system, pituitary hormone preparations, thyroid hormone preparations, antithyroid hormone preparations, and other hormone preparations, agents for the urinary tract, vitamins, hemostatics, anticoagulants, agents for liver disease, antidotes, agents for chemical abuse, agents for the treatment of gout, agents for diabetes, anti-malignant tumor agents, antihistamines, herbs, Chinese medicine, antibiotics, chemotherapeutic agents, insecticides, drugs used to treat protozoal infection, and the like. Specific examples are imipramine, donepazil, diphenhydramine, sumatriptan, naratriptan, eletriptan, rizatriptan, zolmitriptan, almotriptan, flovatriptan, meclofenoxate hydrochloride, chloramphenicol, aminophylline, erythromycin, josamycin, calcium homopantothenate, phenobarbital, cimetidine, famotidine, atorvastatin calcium, etilefrine hydrochloride, diltiazem hydrochloride, propranolol hydrochloride, flufenamic acid; digitoxin, theophylline, promethazine hydrochloride, quinine hydrochloride, sulpyrine, ibuprofen, acetaminophen, ibuprofen [sic], amantadine hydrochloride, oseltamivir phosphate, clarythromycin, acylovir, norfloxacine, sefcapene pivoxil hydrochloride, solifenacin succinate, and nateglinide. One or an appropriate combination of two or more of these drugs can be used. Moreover, these drugs are examples of drugs that can be used in the present invention and should not be interpreted as limiting.

The amount of drug is usually selected as needed in accordance with the type of drug or its medical uses (indications), and there are no special restrictions to the amount of drug as long as it is the amount that is effective for therapy or the amount that is effective for prophylaxis. The amount of drug is 0.5 to 85 wt%, preferably 0.5 to 80 wt%, of the entire coated microparticle. The particularly preferred amount of drug is 0.5 to 70 wt%, and the ideal amount of drug is 0.5 to 50 wt%. The minimum amount of drug of 0.5% usually is not enough for the unpleasant taste of the drug to be noticed. However, there are cases where the unpleasant taste of the drug can be noticed in products containing less than this amount of drug and the above-mentioned amounts should not be interpreted as limiting.

There are no special restrictions to the size of the coated microparticles for quick-disintegrating oral tablets as long as there is not an unpleasant gritty sensation like sand when they are taken contained in quick-disintegrating oral tablets. The average particle diameter is preferably adjusted to 350 µm or smaller. The particularly preferred average particle diameter is 1 to 350 µm, and the ideal average particle diameter is 20 to 350 µm. When the coated microparticles are contained in quick-disintegrating oral tablets, the coated microparticles can account for 0.5 to 75 wt% of the entire quick-disintegrating oral tablets. They preferably account for 1 to 50 wt%, particularly 5 to 40 wt%, of the quick-disintegrating oral tablets.

One or an appropriate combination of two or more conventional additives commonly used in this field can be used in the coated microparticles of the present invention. Examples of this type of additive are binders, disintegrating agents, thickeners, fillers, lubricants, gelling agents, flavoring agents, and fragrances. Examples of binders are polyvinyl pyrrolidone, hydroxypropylmethyl cellulose, hydroxypropyl cellulose, gum arabic powder, gelatin, purulan, polyvinyl alcohol, and pregelatinized starch. Examples of disintegrating agents are corn starch, partly pregelatinized starch, and other starches, carmellose calcium, crospovidone, low-substituted hydroxypropyl cellulose, crystalline cellulose, and croscarmellose sodium. Examples of thickeners are sodium polyacrylate, polyethylene oxide, polycarbophil, hydroxypropylmethyl cellulose, hydroxypropyl cellulose, sodium alginate, propylene glycol alginate, and carrageenan. Examples of lubricants are magnesium stearate, calcium stearate, sucrose fatty acid ester, polyethylene glycol, talc, and stearic acid. Examples of gelling agents are sodium polyacrylate, polyethylene oxide, polycarbophil, hydroxypropylmethyl cellulose, hydroxypropyl cellulose, sodium alginate, mannan, pectin, agar, and carrageenan. Examples of flavoring are aspartame, sucralose, saccharine sodium, dipotassium glycyrrhizinate, stevia, somatin, and citric acid. Examples of fragrances are menthol, peppermint, lemon, lemon-lime, orange, and spearmint oil. These additives are examples and are in no way limiting.

When quick-disintegrating oral tablets are made by mixing the coated microparticles of the present invention with a base of quick-disintegrating oral tablets, the coated microparticles of the present invention can be used as the drug of the conventional quick-disintegrating oral tablets cited in the above-mentioned gazette and quick-disintegrating oral tablets can be made in accordance with the method in this gazette using the base of quick-disintegrating oral tablets cited in this gazette.

Specifically, when the quick-disintegrating oral tablets cited in International Publication 95-20380 are made, the coated microparticles of the present invention are mixed with a sugar of low moldability and then coated and/or granulated using a sugar of high moldability. These granules are tableted and then humidified and dried as necessary to obtain the quick-disintegrating oral tablets. In addition, when the quick-disintegrating oral tablets cited in International Publication Pamphlet 2002-92057 are made, the coated microparticles of the present invention are mixed with diluent and then granulated using a saccharide with a lower melting point than the diluent or the drug that the particles contain. The granules are tableted and heated as needed and made into quick-disintegrating oral tablets.

The coated microparticles of the present invention can be used for purposes other than the above-mentioned quick-disintegrating oral tablets. For instance, they can be used for the molded type of tablets in JP (Kokoku) S62-50445 and JP 2807346; the humidified type in JP (Kokai) H5-271054; the ordinary tableted type in JP H10-182436, JP 3412694, International Publication Pamphlet WO98/02185; and the like. The present invention implies as necessary the technology cited in these patents.

The method for producing the coated microparticles of the present invention will now be described.

The unpleasant-tasting drug itself can serve as the core for producing the coated microparticles of the present invention, but usually drug microparticles that become the core containing the drug are pre-produced. Conventional technology can be used to produce the drug-containing microparticles that serve as the core. For instance, the drug and an appropriate filler (such as microcrystalline cellulose, lactose, or corn starch) are mixed, the mixture is granulated with a binder (such as hydroxypropyl cellulose), and the granules are graded and dried, or a liquid of the drug dissolved or dispersed in a binder and/or film-forming agent solution is sprayed on particles that are an appropriate core (such as microcrystalline cellulose, sucrose granules) to prepare the coated microparticles.

The step whereby coating liquid containing the above-mentioned water-insoluble polymer and water-soluble substance is coated on the prepared drug-containing microparticles consists of the step whereby the coating liquid is prepared and the step whereby the actual coating is performed. The coating liquid is prepared by dissolving or dispersing the water-insoluble polymer and water-soluble substance in water, ethanol, methanol, or another solvent. It goes without saying that these solvents can be used as a mixture if necessary. Coating can be performed using conventional equipment and methods, such as a fluidized bed granulator. The desired coated microparticles for quick-disintegrating oral tablets with a reduced unpleasant taste are obtained by adjusting the amount of coating liquid containing water-insoluble polymer and water-soluble substance on the drug microparticles containing the drug. Side spray film coating is preferably used because coated microparticles that are densely coated and have a narrow particle size distribution free of aggregation can be efficiently produced.

The method for producing quick-disintegrating oral tablets containing the coated microparticles of the present invention will now be described.

The coated microparticles of the present invention are appropriate for mixing with a base of quick-disintegrating oral tablets to make a quick-disintegrating oral tablet. The quick-disintegrating oral tablets cited in International Publication Pamphlet 95-20380, JP (Kokai) H8-19589, JP (Kokai) H9-48726, JP 2919771, JP 3069458, International Publication Pamphlet 2002-92057, and the like are cited as examples of quick-disintegrating oral tablets containing these coated microparticles.

A saccharide is used as the base of quick-disintegrating oral tablets. A saccharide in general, a combination of a saccharide of low moldability and a saccharide of high moldability, a combination of a crystalline saccharide and an amorphous saccharide, a combination of a saccharide with a high melting point and a saccharide with a low melting point, and the like can be selected as needed. For instance, it is possible to mix the coated microparticles of the present invention and the above-mentioned saccharide of low moldability, spray and coat and/or granulate this mixture using a saccharide of high moldability as the binder, and then compression mold the granules.

A saccharide of low moldability means a saccharide that provides a tablet hardness of 0 to 2 kp when, for instance, 150 mg of a saccharide are tableted under tableting pressure of 10 to 50 kg/cm² using a punch with a diameter of 8 mm. A saccharide of high moldability means a saccharide that provides a tablet hardness of 2 kp or greater by the same method. Lactose, mannitol, glucose, sucrose, xylitol, erythritol, or another pharmaceutically acceptable saccharide of low moldability can be used. One or an appropriate combination of two or more saccharides of low moldability can be used. Maltose, maltitol, sorbitol, trehalose, or another pharmaceutically acceptable saccharide of high moldability can be used. One or an appropriate combination of two or more saccharides of high moldability can be used.

It is also possible to mix the coated microparticles of the present invention, a diluent, and a saccharide with a relatively lower melting point than the diluent and the drug contained in the particles, spray and coat and/or granulate this mixture using a binder for quick-disintegrating oral tablets, and then compression mold these granules. This technology can be, for instance, the technology cited in International Publication Pamphlet WO 02/092057, and the "saccharide with a high melting point" and "saccharide with a low melting point" discussed previously and hereafter fall under the definition cited in this literature. The following are examples that can be used.

Xylitol, trehalose, maltose, sorbitol, erythritol, glucose, sucrose, maltitol, and mannitol are examples of pharmaceutically acceptable saccharides with a high melting point. One or an appropriate combination of two or more saccharides with a high melting point can be used. Xylitol, trehalose, maltose, sorbitol, erythritol, glucose, sucrose, maltitol, mannitol, and the like are examples of pharmaceutically acceptable saccharides with a low melting point. One or an appropriate combination of two or more saccharides with a low melting point can be used. Maltitol, copolyvidone, and the like are cited as examples of binders for quick-disintegrating oral tablets. One or an appropriate combination of two or more of these binders can be used.

A humidifying and drying step can be used in order to further increase hardness of the molded tablet that has been prepared. "Humidification" is determined by the apparent critical relative humidity of the saccharide contained in the tablet, but it is usually humidified to critical relative humidity or higher. For instance, humidity is 30 to 100 RH%, preferably 50 to 90 RH%. Temperature in this case is preferably 15 to 50°C, preferably 20 to 40°C. Treatment time is 1 to 36 hours, preferably 12 to 24 hours. There are no limits to the "drying" as long as it is a step whereby the moisture absorbed by humidification is removed. Temperature conditions for drying can be set at, for instance, 10 to 100°C, preferably 20 to 60°C, ideally 25 to 40°C. Treatment time can be 0.5 to 5 hours, and is preferably 1 to 3 hours.

A heating step can also be used in order to improve strength of the prepared molded article when a saccharide of a high melting point and a saccharide of a low melting point are combined. Although determined from the melting point of the saccharide with a low melting point that is contained in the tablets, "heating" is usually heated to a temperature that is the low melting point or higher but less than the high melting point. Treatment time is 0.5 to 120 minutes, preferably 1 to 60 minutes.

### Preferred Embodiments of the Invention

The present invention will now be described while referring to working examples, but the scope of the present invention is not limited to these working examples.

Working Example 1: Preparation of drug-containing coated microparticles for quick-disintegrating oral tablets whose unpleasant taste has been masked (ethyl cellulose/hydroxypropylmethyl cellulose = 75:25 coating)

### [Preparation of drug microparticles]

Imipramine microparticles were obtained by spraying 312.5 g of crystalline cellulose spherical granules (Asahi Kasei Corporation; Celphere CP-102Y) with a mixture of 1,650 g of water and 1,100 g of methanol in which 625.0 g of imipramine hydrochloride and 62.5 g of hydroxypropylmethyl cellulose 2910 (Shin-Etsu Chemical Co., Ltd.; TC-5E) were dissolved at a setting temperature of 85°C, product temperature of 40°C, spraying speed of 12 g/min, and spraying air pressure of 4 kg/cm² using a fluidized bed granulator (Glatt GmbH; GPCG-1).

### [Preparation of coating liquid]

A coating liquid with a combination ratio of water-insoluble polymer ethyl cellulose and water-soluble substance hydroxypropylmethyl cellulose of 75/25 was prepared. 18.9 g of hydroxypropylmethyl cellulose 2910 (Shin-Etsu Chemical Co., Ltd.; TC-5E) were uniformly dissolved in 47.8 g of water. After adding and mixing 1,388.6 g of methanol, 56.7 g of ethyl cellulose were added and uniformly dissolved to obtain the coating solution.

### [Coating]

Drug-containing coated microparticles with a masked taste were obtained by coating 420 g of the above-mentioned imipramine microparticles with the above-mentioned coating solution using a fluidized bed granulator (Freund Industries; UNI-GLATT). The production conditions were a product temperature of 40°C, spraying speed of 4 g/min, and spraying air pressure of 2 kg/cm², and the amount of coating as calculated from the weight of the drug-containing coated microparticles and the weight of the solid component in the weight of the coating liquid that was applied was 10% per drug microparticles. The average particle diameter of the drug-containing coated microparticles was 209 µm.

### Comparative Example 1: Preparation of uncoated microparticles made into uniform matrix form (matrix preparation)

Ten grams of imipramine hydrochloride, 81 g of ethyl cellulose (brand name: Etoxyl, Dow Chemical Company), 9 g of hydroxypropylmethyl cellulose (brand name: TC-5E, Shin-Etsu Chemical Co., Ltd.), 180 g of purified water, and 720 g of methanol were mixed to prepare a solution with a solid concentration of 10 w/w%. This solution was spray dried at a spraying liquid feed rate of 18.5 g/min, inlet temperature of 120°C, and disk rotating speed of 7,500 rpm with a spray dryer (Ohkawara Kakohki Co., Ltd.; L-8) to obtain imipramine matrix particles. The average particle diameter of the imipramine matrix particles at this time was 100 µm.

### Comparative Example 2: Preparation of drug-containing microparticles coated with a base having a different composition ratio (ethyl cellulose/hydroxypropylmethyl cellulose = 9:1 coating)

A coating solution was prepared such that the composition ratio of water-insoluble polymer ethyl cellulose and water-soluble substance hydroxypropylmethyl cellulose was 9/1. Drug microparticles were prepared in accordance with the method of Working Example 1. Drug-containing microparticles were prepared such that the amount of coating was 6%. The average particle diameter of the drug-containing coated microparticles was 213 µm.

### Test 1: Microparticle dissolution test (Working Example 1, Comparative Examples 1 and 2)

The drug-containing coated microparticles obtained in Working Example 1 were weighed out such that the amount of imipramine hydrochloride was 50 mg and dissolution tests were conducted in accordance with Japanese Pharmacopoeia Dissolution Testing Method No. 2 using an automatic 6-series dissolution tester (Toyama Sangyo Co., Ltd.) (Table 1). Tests were similarly conducted on the microparticles obtained in Comparative Examples 1 and 2. The test fluids were 500 mL of phosphate buffer with a pH of 6.8 (Japanese Pharmacopoeia Disintegration Testing Methods Fluid No. 2) and 500 mL of a hydrochloride buffer with a pH of 1.2 (Japanese Pharmacopoeia Disintegration Testing Methods Fluid No. 1). The paddle rotating speed was 100 rpm.

**Table 1. Results of microparticle dissolution tests (%)**

| | pH 6.8 | | pH 1.2 |
|---|---|---|---|
| | 1 min | 30 min | 30 min |
| Working Example 1 | 3.3% | 81.6% | 84.9% |
| Comparative Example 1 | 31.5% | 58.2% | ---- |
| Comparative Example 2 | 12.7% | 79.7% | ----- |

### Results and Discussion

Drug dissolution from the coated microparticles obtained in Working Example 1 showed sufficient control of initial drug dissolution at 3.3% after one minute, as shown in Table 1, and fast drug dissolution thereafter regardless of pH at 81.6% (pH of 6.8) and 84.9% (pH of 1.2) after 30 minutes. Consequently, it was inferred that the pharmaceutical preparation of the present invention makes it possible to simultaneously realize sufficient control drug dissolution in the oral cavity and excellent drug dissolution in gastrointestinal tract regardless of pH.

On the other hand, dissolution of the drug could not be sufficiently controlled and was 31.5% after one minute with the matrix pharmaceutical preparation of Comparative Example 1. Consequently, it was inferred that when imipramine is made into matrix-type microparticles, the unpleasant taste thereof cannot be reduced.

Moreover, drug dissolution from the coated microparticles obtained in Comparative Example 2, wherein the composition ratio of water-insoluble polymer ethyl cellulose and water-soluble substance hydroxypropylmethyl cellulose was 9/1 and the amount of coating was 6%, did not show sufficient control of initial drug dissolution at 12.7% after one minute. Based on the above-mentioned, drug-containing microparticles produced bringing the composition ratio of water-insoluble polymer and water-soluble substance to 9/1 cannot simultaneously realize sufficient control of drug dissolution in the oral cavity and excellent drug dissolution in gastrointestinal tract.

Furthermore, when attention is focused on drug dissolution 30 minutes after starting dissolution tests, it is clear that the drug-containing microparticles obtained in Working Example 1 realize fast drug dissolution even though the amount of coating of the coating layer is greater than that in Comparative Example 2. This indicates that by means of the present invention, it is possible to realize sufficient control of drug dissolution in the oral cavity as well as fast drug dissolution in gastrointestinal tract.

Working Example 2: Preparation of quick-disintegrating oral tablets containing coated microparticles

A mixture of 247.6 g of mannitol (Towa Kasei; Mannito P) that had been pulverized with a pin mill (Hosokawa Micron Corporation; Fine Impact Mill 100UPZ) and screened with a 20-mesh sieve and 60.0 g of the drug-containing coated microparticles obtained in Working Example 1 were granulated with an aqueous solution containing 62.5 g of maltose (Hayashibara Co., Ltd.; Sunmalt S) using a fluidized bed granulator (Glatt GmbH; GPCG-1) to obtain granulated particles for quick-disintegrating oral tablets.

Then 14.4 mg of peppermint flavor (T. Hasegawa Co., Ltd.), 14.4 mg of aspartame (Ajinomoto Co., Inc.), and 3.0 mg of magnesium stearate (Merck & Co., Inc.) were mixed with 568.2 mg of the above-mentioned granulated particles, the mixture was filled into dies with a diameter of 13 mm, and tablets were made by tableting at a pressure of 1.25 KN using an autograph (Shimadzu Corporation; AGS-20KNG). These tablets were stored for 18 hours under heating and humidification at 25°C/75% RH using a thermohydrostatic chamber (Tabaiespec Co. Ltd.; PR-35C). Then they were dried for three hours at 30°C (humidity of 40%) to obtain quick-disintegrating oral tablets. Hardness of the resulting tablets obtained was 7.9 kp (n = 3).

Working Example 3: Preparation of quick-disintegrating oral tablets containing coated microparticles

A mixture of 247.6 g of mannitol (Towa Kasei; Mannito P) that had been pulverized with a pin mill (Hosokawa Micron Corporation; Fine Impact Mill 100UPZ) and screened with a 20-mesh sieve, 32.1 g of erythritol (Nikken Chemicals Co., Ltd.) also screened with a 20-mesh sieve, and 60.0 g of the drug-containing coated microparticles obtained in Working Example 1 was granulated with an aqueous solution containing 8.0 g of copolyvidone (BASF AG; Kollidon VA64) to obtain granulated particles for quick-disintegrating oral tablets.

Then 14.4 mg of peppermint flavor (T. Hasegawa Co., Ltd.), 14.4 mg of aspartame (Ajinomoto Co., Inc.), and 3.0 mg of magnesium stearate (Merck & Co., Inc.) were mixed with 568.2 mg of the above-mentioned granulated particles, the mixture was filled into dies with a diameter of 13 mm, and tablets were made by tableting at a pressure of 2.0 KN using an autograph (Shimadzu Corporation; AGS-20KNG). These tablets were heated for ten minutes at 122°C using a program oven (model MOV-112P, Sanyo) and then allowed to cool naturally at room temperature for 30 minutes to obtain quick-disintegrating oral tablets. Hardness of the resulting tablets was 17.2 kp (n = 3).

### Comparative Example 3: Preparation of quick-disintegrating oral tablets having drug-containing microparticles coated with base of different composition (ethyl cellulose/hydroxypropylmethyl cellulose = 9:1 coating)

A mixture of 278.3 g of mannitol (Towa Kasei; Mannito P) that had been pulverized with a pin mill (Hosokawa Micron Corporation, Fine Impact Mill 100UPZ) and screened with a 20-mesh sieve and 60.0 g of the drug-containing coated microparticles coated with a base of a different composition ratio obtained in Comparative Example 2 was granulated with an aqueous solution containing 63.7 g of maltose (Hayashibara Co., Ltd.; Sunmalt S) to obtain granulated particles for quick-disintegrating oral tablets.

Then 14.4 mg of peppermint flavor (T. Hasegawa Co., Ltd.), 14.4 mg of aspartame (Ajinomoto Co., Inc.), and 3.0 mg of magnesium stearate (Merck & Co., Inc.) were mixed with 568.2 mg of the above-mentioned granulated particles, the mixture was filled into dies with a diameter of 13 mm, and tablets were made by tableting at a pressure of 1.25 KN using an autograph (Shimadzu Corporation; AGS-20KNG). These tablets were stored for 18 hours under heating and humidification at 25°C/75% RH using a thermohydrostatic chamber (Tabaiespec Co., Ltd.; PR-35C). Then they were dried for three hours at 30°C (humidity of 40%) to obtain quick-disintegrating oral tablets.

### Test 2: Dissolution tests on quick-disintegrating oral tablets

Dissolution tests were conducted on the quick-disintegrating oral tablets obtained in Working Examples 2 and 3 in accordance with Japanese Pharmacopoeia Dissolution Testing Method No. 2 using an automatic 6-series dissolution tester (Toyama Sangyo Co., Ltd.) (Table 2). The test fluid was 500 mL of phosphate buffer with a pH of 6.8 (Japanese Pharmacopoeia Disintegration Testing Methods Fluid No. 2). The paddle rotating speed was 100 rpm.

**Table 2. Results of quick-disintegrating oral tablets dissolution tests (%)**

| | 1 min | 30 min |
|---|---|---|
| Working Example 2 | 4.3% | 81.0% |
| Working Example 3 | 8.1% | 89.5% |

### Results and Discussion

Drug dissolution from the quick-disintegrating oral tablets obtained in Working Examples 2 and 3 showed sufficient control of initial drug dissolution at 4.3% and 8.1 % after one minute and fast dissolution thereafter at 81.0% and 89.5% after 30 minutes. Consequently, as with the results from the drug-containing coated microparticles obtained in Working Example 1 shown in Table 1, it was inferred that these are quick-disintegrating oral tablets with which sufficient control of oral drug dissolution and excellent gastrointestinal drug dissolution can be simultaneously realized.

### Test 3: Sensory tests of quick-disintegrating oral tablets

Three healthy adults were administered the quick-disintegrating oral tablets obtained in Working Example 2 and Comparative Example 3 and sensory tests were conducted (Table 3).

**Table 3. Sensory test results of quick-disintegrating oral tablets**

| | Subject | Oral disintegration time | Unpleasant taste (1 minute after administration | Unpleasant taste (5 minutes after administration) |
|---|---|---|---|---|
| Working Example 2 | No. 1 | 30 seconds | ± | - |
| | No. 2 | 32 seconds | - | - |
| | No. 3 | 36 seconds | ± | ± |
| | Average | 33 seconds | ---- | ---- |
| Comparative Example 3 | No. 1 | 30 seconds | ++ | + |
| | No. 2 | 28 seconds | ++ | + |
| | No. 3 | 35 seconds | + | + |
| | Average | 31 seconds | ---- | ---- |

Evaluation of unpleasant taste: - (Did not notice an unpleasant taste); ± (Noticed slight change in taste, but was not unpleasant); + (Noticed an unpleasant taste), ++ (Noticed a strong unpleasant taste)

### Results and Discussion

As shown in Table 3, the oral disintegration time of the quick-disintegrating oral tablets prepared in Working Example 2 was 33 seconds (n = 3) and virtually no unpleasant taste was noticed. Consequently, it is clear that the quick-disintegrating oral tablets containing the coated microparticles prepared by the present invention showed properties that were sufficient for quick-disintegrating oral tablets.

On the other hand, when attention is focused on an unpleasant taste after taking the tablets, only a slight change in taste was noticed with the quick-disintegrating oral tablets prepared in Working Example 2, while a strong unpleasant taste was noticed one minute after administration of the quick-disintegrating oral tablets prepared in

### Comparative Example 3.

Moreover, it is clear from Table 1 that in contrast to the fact that drug dissolution from the drug-containing coated microparticles prepared in Working Example 1 is 3.3% after one minute, drug dissolution from drug-containing particles with a different composition ratio prepared in Comparative Example 2 was 12.7% after one minute.

That is, according to the above-mentioned sensory tests and results of drug-containing coated microparticle dissolution tests, drug dissolution in the oral cavity is not sufficiently controlled with the drug-containing coated microparticles prepared in Comparative Example 2; therefore, it can be said that one-minute dissolution must be kept to 10% or less in order to control the unpleasant taste of a drug.

Working Example 4: Preparation of drug-containing coated microparticles for quick-disintegrating oral tablets with a masked unpleasant taste (drug: diphenhydramine hydrochloride, coating film composition: ethyl cellulose/hydroxypropylmethyl cellulose = 75:25 coating)

### [Preparation of drug microparticles]

400.0 g of crystalline cellulose spherical granules (Asahi Kasei Corporation; Celphere SCP 100) were measured out and sprayed with a mixture of 850 g of water and 850 g of methanol in which 200.0 g of diphenhydramine hydrochloride (Kongo Chemical Co., Ltd: Japanese Pharmacopoeia) and 100.0 g of hydroxypropylmethyl cellulose 2910 (Shin-Etsu Chemical Co., Ltd.; TC-5E) had been dissolved at a setting temperature of 80°C, product temperature of 42°C, spraying speed of 8 g/min, and spraying air pressure of 3 kg/cm² using a fluidized bed granulator (Freund Industries; UNI-GLATT) to obtain diphenhydramine hydrochloride microparticles.

### [Preparation of coating liquid]

A coating liquid with a composition ratio of water-insoluble polymer ethyl cellulose and water-soluble substance hydroxypropylmethyl cellulose of 75/25 was prepared. 22.5 g of hydroxypropylmethyl cellulose 2910 (Shin-Etsu Chemical Co., Ltd.; TC-5E) were uniformly dissolved in 68.4 g of water. After adding and mixing 1,641.6 g of methanol, 67.5 g of ethyl cellulose were added and uniformly dissolved to obtain the coating solution.

### [Coating]

Drug-containing coated microparticles with a masked taste were obtained by coating 300 g of the above-mentioned diphenhydramine hydrochloride microparticles with the above-mentioned coating solution using a fluidized bed granulator (Freund Industries; UNI-GLATT). The production conditions were a setting temperature of 63°C, product temperature of 37°C, spraying speed of 5 g/min, and spraying air pressure of 3 kg/cm², and the amount of coating as calculated from the weight of the drug-containing coated microparticles and the weight of the solid component in the weight of the coating liquid that was applied was 30% per drug microparticles. The average particle diameter of the drug-containing coated microparticles was 150 µm.

Working Example 5: Preparation of drug-containing coated microparticles for' quick-disintegrating oral tablets with a masked unpleasant taste (drug: acetaminophen, coating film composition: ethyl cellulose/hydroxypropylmethyl cellulose = 75:25 coating)

### [Preparation of drug microparticles]

500.0 g of crystalline cellulose spherical granules (Asahi Kasei Corporation; Celphere CP-102Y) were measured out and sprayed with a mixture of 220 g of water and 880 g of methanol in which 250.0 g of acetaminophen (Yoshitomi Fine Chemicals, Ltd., Japanese Pharmacopoeia) and 25.0 g of hydroxypropylmethyl cellulose 2910 (Shin-Etsu Chemical Co., Ltd.; TC-5E) were dissolved at a setting temperature of 70°C, product temperature of 31 °C, spraying speed of 8 g/min, and spraying air pressure of 3 kg/cm² using a fluidized bed granulator (Freund Industries; UNI-GLATT) to obtain acetaminophen microparticles.

### [Preparation of coating liquid]

A coating liquid with a composition ratio of water-insoluble polymer ethyl cellulose and water-soluble substance hydroxypropylmethyl cellulose of 75/25 was prepared. 15.0 g of hydroxypropylmethyl cellulose 2910 (Shin-Etsu Chemical Co., Ltd.; TC-5R) were uniformly dissolved in 68.4 g of water. After adding and mixing 1,071.6 g of methanol, 45.0 g of ethyl cellulose were added and uniformly dissolved to obtain the coating solution.

### [Coating]

Drug-containing coated microparticles with a masked taste were obtained by coating 300 g of the above-mentioned acetaminophen microparticles with the above-mentioned coating solution using a fluidized bed granulator (Freund Industries; UNI-GLATT). The production conditions were a setting temperature of 63°C, product temperature of 33°C, spraying speed of 5 g/min, and spraying air pressure of 3 kg/cm², and the amount of coating as calculated from the weight of the drug-containing coated microparticles and the weight of the solid component in the weight of the coating liquid that was applied was 16% per drug microparticles. The average particle diameter of the drug-containing coated microparticles was 163 µm.

Working Example 6: Preparation of drug-containing coated microparticles for quick-disintegrating oral tablets with a masked taste (drug: acetaminophen, coating film composition: ethyl cellulose/hydroxypropyl cellulose = 75:25 coating)

### [Preparation of coating liquid]

A coating liquid with a composition ratio of water-insoluble polymer ethyl cellulose and water-soluble substance hydroxypropyl cellulose of 75/25 was prepared. After adding and dissolving 1,140.0 g of methanol into 15.0 g of hydroxypropyl cellulose (Nippon Soda Co., Ltd.; HPC-SL), 45.0 g of ethyl cellulose were added and uniformly dissolved to obtain the coating solution.

### [Coating]

Drug-containing coated microparticles with a masked taste were obtained by coating 300 g of the acetaminophen microparticles obtained in Working Example 5 with the above-mentioned coating solution using a fluidized bed granulator (Freund Industries; UNI-GLATT). The production conditions were a setting temperature of 53°C, product temperature of 37°C, spraying speed of 5 g/min, and spraying air pressure of 3 kg/cm², and the amount of coating as calculated from the weight of the drug-containing coated microparticles and the weight of the solid component in the weight of the coating liquid that was applied was 18% per drug microparticles. The average particle diameter of the drug-containing coated microparticles was 163 µm.

### Test 4: Microparticle dissolution test (Working Examples 4, 5, 6)

The drug-containing coated microparticles obtained in Working Example 4 were weighed out such that the amount of diphenhydramine hydrochloride was 50 mg, while the drug-containng coated microparticles obtained in Working Examples 5 and 6 were weighed out such that the amount of acetaminophen was 20 mg, and dissolution tests were conducted in accordance with Japanese Pharmacopoeia Dissolution Testing Method No. 2 using an automatic 6-series dissolution tester (Toyama Sangyo Co., Ltd.) (Table 4). The test fluid was 500 mL of phosphate buffer with a pH of 6.8 (Japanese Pharmacopoeia Disintegration Testing Methods Fluid No. 2). The paddle rotating speed was 100 rpm.

**Table 4. Results of microparticle dissolution tests (%)**

| | pH 6.8 | |
|---|---|---|
| | 1 min | 30 min |
| Working Example 4 | 4.9% | 88.3% |
| Working Example 5 | 2.9% | 99.7% |
| Working Example 6 | 3.1 % | 99.8% |

### Results and Discussion

Sufficient control of early drug dissolution and thereafter fast drug dissolution were realized from the coated microparticles obtained in Working Examples 4, 5, and 6, with drug dissolution after one minute being 4.9%, 2.9%, and 3.1%, respectively, and drug dissolution after 30 minutes being 88.3%, 99.7%, and 99.8%, respectively, as shown in Table 1. Consequently, it can be inferred that the preparation of the present invention makes it possible to simultaneously realize sufficient control of oral drug dissolution and excellent gastrointestinal drug dissolution.

Working Example 7: Preparation of drug-containing coated microparticles for quick-disintegrating oral tablets with a masked unpleasant taste (drug: imipramine hydrochloride, coating film composition: ethyl cellulose/hydroxypropylmethyl cellulose = 70:30 coating)

### [Preparation of drug microparticles]

300.0 g of crystalline cellulose spherical granules (Asahi Kasei Corporation; Celphere CP-102Y) were measured out and sprayed with a solution of 2,640 g of water in which 600.0 g of imipramine hydrochloride and 60.0 g of hydroxypropylmethyl cellulose 2910 (Shin-Etsu Chemical Co., Ltd.; TC-5E) were dissolved at a setting temperature of 50°C, product temperature of 25°C, spraying speed of 8 g/min, and spraying air pressure of 3 kg/cm² using a fluidized bed granulator (Glatt GmbH; GPCG-1) to obtain imipramine microparticles.

### [Preparation of coating liquid]

A coating liquid with a composition ratio of water-insoluble polymer ethyl cellulose and water-soluble substance hydroxypropylmethyl cellulose of 70/30 was prepared. 36.0 g of hydroxypropylmethyl cellulose 2910 (Shin-Etsu Chemical Co., Ltd.; TC-5E) were uniformly dissolved in 108.0 g of water. After adding and mixing 2,172.0 g of methanol, 84.0 g of ethyl cellulose were added and uniformly dissolved to obtain the coating solution.

### [Coating]

Drug-containing coated microparticles with a masked taste were obtained by coating 300 g of the above-mentioned imipramine microparticles with the above-mentioned coating solution using a fluidized bed granulator (Glatt, GmbH; GPCG-1). The production conditions were a setting temperature of 60°C, product temperature of 30°C, spraying speed of 5.5 g/min, and spraying air pressure of 2 kg/cm², and the amount of coating as calculated from the weight of the drug-containing coated microparticles and the weight of the solid component in the weight of the coating liquid that was applied was 30% per drug microparticles.

Working Example 8: Preparation of drug-containing coated microparticles for quick-disintegrating oral tablets with a masked unpleasant taste (drug: imipramine hydrochloride, coating film composition: ethyl cellulose/hydroxypropylmethyl cellulose = 60:40 coating)

### [Preparation of coating liquid]

A coating liquid with a composition ratio of water-insoluble polymer ethyl cellulose and water-soluble substance hydroxypropylmethyl cellulose of 60/40 was prepared. 60.0 g of hydroxypropylmethyl cellulose 2910 (Shin-Etsu Chemical Co., Ltd.; TC-5E) were uniformly dissolved in 180.0 g of water. After adding and mixing 2,670.0 g of methanol, 90.0 g of ethyl cellulose were added and uniformly dissolved to obtain the coating solution. More of the same solution was separately prepared.

### [Coating]

Drug-containing coated microparticles with a masked taste were obtained by coating 300 g of the imipramine microparticles obtained in Working Example 7 with the above-mentioned coating solution using a fluidized bed granulator (Glatt, GmbH; GPCG-1). The production conditions were a setting temperature of 65°C, product temperature of 36°C, spraying speed of 5.5 g/min, and spraying air pressure of 2 kg/cm², and the amount of coating as calculated from the weight of the drug-containing coated microparticles and the weight of the solid component in the weight of the coating liquid that was applied was 57% and 80% per drug-containing microparticles.

### Test 5: Microparticle dissolution tests (Working Examples 7, 8)

The drug-containing coated microparticles obtained in Working Examples 7 and 8 were weighed out such that the amount of imipramine hydrochloride was 50 mg, and dissolution tests were conducted in accordance with Japanese Pharmacopoeia Dissolution Testing Method No. 2 using an automatic 6-series dissolution tester (Toyama Sangyo Co., Ltd.) (Table 5). The test fluid was 500 mL of phosphate buffer with a pH of 6.8 (Japanese Pharmacopoeia Disintegration Testing Methods Fluid No. 2). The paddle rotating speed was 100 rpm.

**Table 5. Results of microparticle dissolution tests (%)**

| | pH 6.8 | |
|---|---|---|
| | 1 min | 30 min |
| Working Example 7 | 3.2% | 87.1 % |
| Working Example 8 (57%) | 9.8% | 98.8% |
| Working Example 8 (80%) | 0.4% | 95.9% |

### Results and Discussion

Sufficient control of early drug dissolution and thereafter fast drug dissolution were realized from the coated microparticles obtained in Working Examples 7 and 8 with drug dissolution after one minute being 3.2%, 9.8%, and 0.4%, respectively, and drug dissolution after 30 minutes being 87.1%, 98.8%, and 95.9%, respectively, as shown in Table 5. Consequently, it can be inferred that the preparation of the present invention makes it possible to simultaneously realize sufficient control of oral drug dissolution and excellent gastrointestinal drug dissolution. On the other hand, it was evaluated that in Working Example 8, coating of 57% or more was necessary in order to realize "fast gastrointestinal drug dissolution," that is, a drug dissolution rate of 80% or higher 30 minutes after starting the drug dissolution test. Although it does not appear that there are any particular problems with production when this amount of coating is used, it is easily inferred that the coating percentage needed to realize the above-mentioned dissolution rate will increase if the percentage of water-soluble substance increases beyond this point; therefore, there is a concern that there will be a reduction in productivity because of prolonged production steps. Consequently, it was considered that 40% or less is the appropriate percentage of the water-soluble substance.

### Industrial Applicability

The present invention is useful for unpleasant-tasting drugs that have high water solubility in that it is possible to provide drug-containing coated microparticles for quick-disintegrating oral tablets with which sufficient control of oral drug dissolution and fast gastrointestinal drug dissolution are simultaneously satisfied, and with which an unpleasant taste can be sufficiently controlled by coating microparticles containing a drug with a film that is a combination at a specific ratio of a specific water-insoluble polymer and water-soluble substance.

Consequently, the drug-containing coated microparticles of the present invention are promising in that even if they are used for a drug with high water solubility and a very low threshold value, the unpleasant taste of the drug can be sufficiently reduced without any reduction in bioavailability, and in that they can be easily produced by equipment of different scales.

## Claims

1. Drug-containing coated microparticles for quick-disintegrating oral tablets wherein microparticles containing a drug with an unpleasant taste are coated with a film composed of
(1) a pH-independent water-insoluble polymer accounting for 60% or more but less than 80% of the film and
(2) a pH-independent water-soluble substance accounting for more than 20% to 40% or less of the film,
these microparticles being **characterized in that** the rate of dissolution of the drug from the drug-containing microparticles is 0% to 10% in one minute and 80% to 100% in 30 minutes, and the average particle diameter is 350 µm or less.

2. The drug-containing coated microparticles for quick-disintegrating oral tablets according to claim 1, **characterized in that** the pH-independent water-insoluble polymer is one or more selected from the group consisting of ethyl cellulose, aminoalkyl methacrylate copolymer RL, aminoalkyl methacrylate copolymer RS, and ethyl acrylate-methyl methacrylate copolymer.

3. The drug-containing coated microparticles for quick-disintegrating oral tablets according to claim 1, **characterized in that** the pH-independent water-soluble substance is one or more selected from the group consisting of hydroxypropylmethyl cellulose, hydroxypropyl cellulose, hydroxyethyl cellulose, polyvinyl pyrrolidone, polyethylene glycol, polyethylene oxide, lactose, sucrose, mannitol, and maltose.

4. The quick-disintegrating oral tablets containing drug-containing coated microparticles for quick-disintegrating oral tablets according to claim 1.
